# EUROPEAN PATENT SPECIFICATION

(11) **EP 1 423 151 B1**
(45) Date of publication and mention of the grant of the patent: **31.12.2008**
(21) Application number: 01968193.1
(22) Date of filing: 28.08.2001
(51) Int. Cl.: A61K 49/00

(54) **LIGHT-STABILIZED IN VIVO STAIN COMPOSITION AND METHOD OF MANUFACTURE**
MIT LICHT STABILISIERTE (IN VIVO) FÄRBEZUSAMMENSETZUNG UND HERSTELLUNGSVERFAHREN
COMPOSITION COLORANTE I IN VIVO /I STABLE A LA LUMIERE ET PROCEDE DE FABRICATION

(43) Date of publication of application: 02.06.2004
(73) Proprietor: Zila Biotechnology, Inc., Phoenix, AZ 85014-2800 (US)
(72) Inventor: BURKETT, Douglas, D., Phoenix, AZ 85044 (US)
(74) Representative: Brereton, Paul Arthur
(86) International application number: PCT/US2001/026805
(87) International publication number: WO 2003/020323

(56) References cited:
- WO-A-99/25388
- US-A- 6 086 852
- HAVELCOVA M. ET AL.: 'Photophysical properties of thiazine dyes in aqueous solution and in micelles' DYES AND PIGMENTS vol. 44, 2000, pages 49 - 54, XP002906581

## Description

This invention relates to light-stabilized thiazine dye biological stain compositions, illustratively, the tolonium chloride ("TC") dye compositions disclosed in my United States Patent No. 6,086,852.

In another respect the invention concerns methods of manufacturing such compositions.

In particular the invention contemplates light-stabilized tolonium chloride ("TC") dye compositions and their manufacture.

### Background of the Invention

As far as known, prior workers, such as Mashberg (U.S. Patent 4,321,251), who instigated the use of TC for *in vivo* identification of dysplasia, used prior art dye products in which the conformational isomers of TC plus the N-demethylation and N,N-demethylation derivatives of the conformational isomers were less than 80% of the dye composition and in which the two N-demethylation derivatives of the conformational isomers, formed greater than about 20% % of the dye composition. According to my information, prior workers were unaware of the exact composition of their "TC" products and manufacturers of prior art TC products were unable to reproducibly prepare them. In fact, the prevalent literature description of the quality of TC was simply that it have "good color value". The Biological Stain Commission specifies an analytical titration procedure for determining only the "organic dye content" of the TC material. The prior art use of such loosely defined "TC." resulted in anomalous clinical observations and serious problems in obtaining necessary regulatory clearances to manufacture and market such products for use in human diagnostic procedures.

In addition to the problem of variable initial composition, prior art TC and other thiazine biological stains were subject to time-related variations in composition. For example, Dean et al. in "Stains Technology", Vol. 32, No. 1, pp. 35 et seq. (1977) recommended that thiazine dyes in methanolic solutions should be refrigerated to prevent further oxidative N-demethylation. Liao, et al. reported in "Stains Technology", Vol. 57, No. 1, pp. 23 et seq. (1982) that reduction in methylene blue content by precipitation from methanolic Wright's stain solution could be markedly decreased by simultaneous addition of diethylamine hydrochloride and dimethylamine hydrochloride.

In my United States Patent No. 6,086,852, I describe a process for reproducibly manufacturing high-quality TC products initially having a high proportion of the the conformational isomers with respect to the N-demethylation products of such isomers. While exclusion of contact with air and avoidance of high temperatures retard oxidative N-demethylation of the conformational isomers, it is observed that, in the absence of free radical scavengers, such as metal ions, alcoholic solutions of this TC product undergo light-induced or photochemical N-demethylation.

### Brief Description of the Invention

I have now discovered improvements in solutions of thiazine dyes, wherein molecules of the dye act as both the sensitizers and substrates in photochemical oxidative N-demethylation reactions, in which reactions some of said dye molecules absorb light and are converted to the singlet state, some of said singlet-state dye molecules react with unactivated substrate dye molecules to form triplet state molecules by intersystem crossing, some of said triplet-state molecules react with ambient ground-state oxygen to produce singlet-state oxygen molecules, and some of said singlet-state oxygen molecules demethylate ground-state dye molecules. My improvements, which substantially reduce said demethylation comprises quenching at least some of the triplet-state dye, preferably by incorporating into said solution a free radical scavenger, e.g., a zinc ion, thus returning the triplet-state dye molecules to the unreactive ground-state.

### Detailed Description of the Invention

Oxidative N-demethylation of a thiazine dye is envisioned to occur as illustrated below for the demethylation of TC: 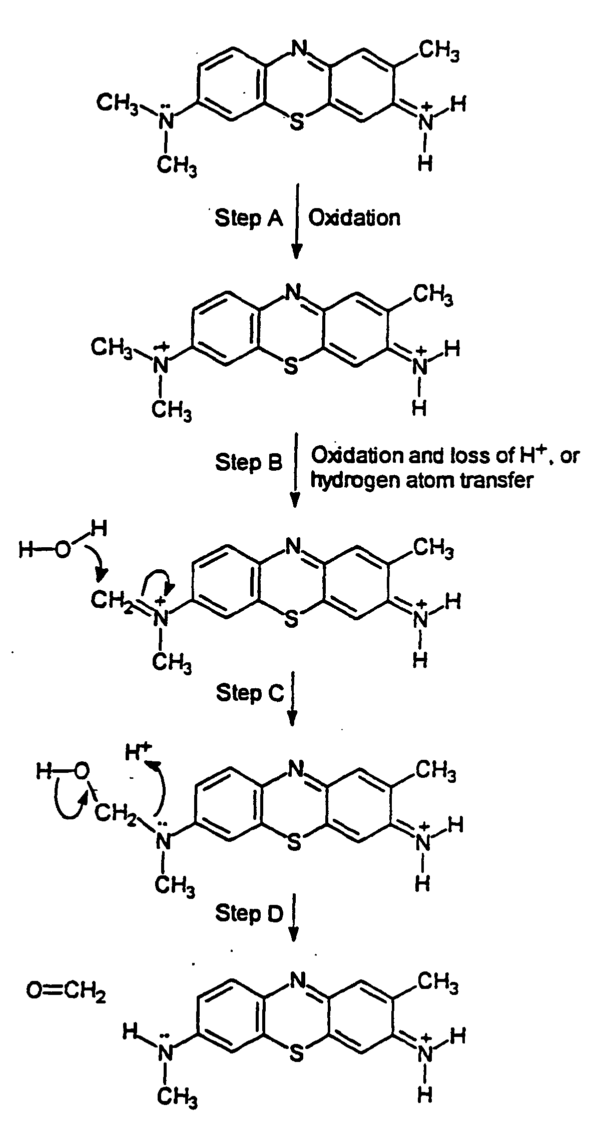 In Steps A and B, an electron is removed from the TC to produce a radical cation, which is then oxidized again and loses a proton to form an iminium ion. Subsequently, the iminium ion undergoes nucleophilic attack by water (Step C) to produce the carbinolamine intermediate, in which there is a hydroxyl group and an amino group attached to the same carbon atom. The carbinolamine is unstable, and loss of the amine (Step D) liberates formaldehyde and monodemethylated TC. In the case of demethylation of methylene blue, this would correspond to the formation of Azure C from Azure A.

In photochemical demethylation reactions, TC apparently acts both as the sensitizer and the substrate in photochemical reactions, i.e., in the absence of free-radical scavengers, it brings about its own photodecomposition. Thus, TC absorbs light and becomes a "photosensitizer" and brings about a direct reaction of the TC in its singlet excited state with another TC molecule "substrate." The substrate molecule reacts with the triplet state of the dye, formed by intersystem crossing (isc). Singlet oxygen is formed by reaction of ground state oxygen with the triplet state of the dye, followed by reaction of singlet oxygen with the substrate. 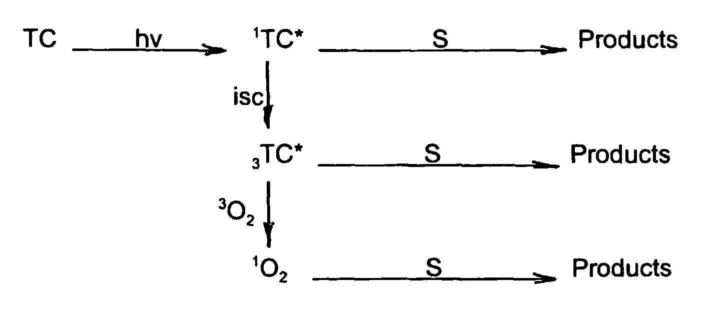 The "heavy atom" effect of a metal ion, such as zinc, sodium, and the like, quenches the triplet state of the dye, returning it to is unreactive ground singlet state, preventing the triplet state from participation in the degradation reactions.

The amounts of metal ion required to provide high photochemical stability can be determined by routine experimentation having regard for this disclosure. In general, I prefer to employ upward of about 4 wt.% zinc optionally with 9 wt.% sodium in the dye solution, although greater amounts are not harmful and smaller amounts are at least partially effective. The zinc content should be a minimum of upwards of 2 wt.% for at least partial effectiveness.

### EXAMPLES

### EXAMPLE 1

This example illustrates the light-instability of a TC product with negligible content of metal ions.

A sample of the TC product is prepared in accordance with Example 1 of my United States Patent 6,086,852, and further purified by preparative high performance liquid chromatography. The total metal content of this sample is less than 0.2 wt. %.

An aliquout of this purified TC product sample is deep-blue in color. After standing overnight in the laboratory, exposed to ambient incandescent light, the solution becomes colorless.

### EXAMPLE 2

The sample of Example 1 except containing about 4 wt% Zn (as zinc chloride) and 9% Na (as sodium chloride) in the solution is essentially completely stable in the presence of incandescent light.

### EXAMPLE 3

The procedures of Examples 1 and 2 are repeated using methylene blue, Azure A and Azure C instead of TC. Similar results are obtained.

### EXAMPLE 4

The procedures of Example 1-3 are repeated except using only zinc chloride in solution and only sodium chloride in solution. Equivalent results are obtained.

### EXAMPLE 5

The procedures of Example 1-4 are repeated except that soluble salts of magnesium, chromium and silicon are used instead of zinc and sodium salts. Similar results are obtained.

## Claims

1. A solution of a thiazine dye, wherein molecules of said dye act as both the sensitizers and substrates in photochemical oxidative demethylation reactions, in which reactions
some of said dye molecules absorb light and are converted to the singlet state,
some of said singlet-state dye molecules react with unactivated substrate dye molecules to form triplet state molecules by intersystem crossing,
some of said triplet-state molecule react with ambient ground-state oxygen to produce singlet-state oxygen molecules, and
some of said singlet-state oxygen molecules demethylate ground-state dye molecules;
comprising upwards of 2 wt.% metal ions for substantially reducing said demethylation reactions by quenching some of said triplet-state dye molecules, returning them to the unreactive ground-state, wherein the metal ions are zinc ions.

2. A solution according to claim 1 comprising upwards of 4 wt.% zinc ions.

3. A solution according to claim 1 or claim 2 further comprising sodium ions.

4. A solution according to any preceding claim further comprising upwards of 9 wt.% sodium ions.

## Patentansprüche

1. Lösung aus einem Thiazinfarbstoff, wobei Moleküle des genannten Farbstoffs sowohl als Sensibilisatoren als auch als Substrate in fotochemischen oxydativen Demethylierungsreaktionen dienen, wobei in den Reaktionen
einige der genannten Farbstoffmoleküle Licht absorbieren und in den Singulettzustand umgewandelt werden,
einige der genannten Farbstoffmoleküle im Singulettzustand mit nicht aktivierten Substratfarbstoffmolekülen reagieren, um Moleküle im Triplettzustand durch Zwischensystemübergang zu bilden,
einige der genannten Moleküle im Triplettzustand mit Sauerstoff im Umgebungsgrundzustand reagieren, um Sauerstoffmoleküle im Singulettzustand zu produzieren, und
einige der genannten Sauerstoffmoleküle im Singulettzustand Farbstoffmoleküle im Grundzustand demethylieren,
umfassend mehr als 2 Gew.-% Metallionen, um die genannten Demethylierungsreaktionen durch Abschrecken einiger der genannten Farbstoffmoleküle im Triplettzustand im Wesentlichen zu reduzieren, wobei sie in den reaktionsunfähigen Grundzustand zurückgeführt werden, wobei die Metallionen Zinkionen sind.

2. Lösung nach Anspruch 1, die mehr als 4 Gew.-% Zinkionen beinhaltet.

3. Lösung nach Anspruch 1 oder Anspruch 2, die ferner Natriumionen beinhaltet.

4. Lösung nach einem der vorherigen Ansprüche, die ferner mehr als 9 Gew.-% Natriumionen umfasst.

## Revendications

1. Solution de colorant thiazine, dans laquelle des molécules dudit colorant agissent à la fois en tant que sensibilisateurs et substrats dans des réactions de déméthylation oxydante photochimique, dans lesquelles réactions
certaines desdites molécules de colorant absorbent la lumière et sont converties en l'état singulet,
certaines desdites molécules de colorant à l'état singulet réagissent avec des molécules de colorant de substrat non activées afin de former des molécules à l'état triplet par croisement intersystèmes,
certaines desdites molécules à l'état triplet réagissent avec l'oxygène à l'état fondamental ambiant afin de produire des molécules d'oxygène à l'état singulet, et
certaines desdites molécules d'oxygène à l'état singulet déméthylent les molécules de colorant à l'état fondamental ;
comprenant plus de 2 % en poids d'ions métalliques pour considérablement réduire lesdites réactions de déméthylation en piégeant certaines des molécules de colorant à l'état triplet, les ramenant à l'état fondamental non réactif, dans laquelle les ions métalliques sont des ions de zinc.

2. Solution selon la revendication 1, comprenant plus de 4 % en poids d'ions de zinc.

3. Solution selon la revendication 1 ou la revendication 2, comprenant en outre des ions de sodium.

4. Solution selon l'une quelconque des revendications précédentes, comprenant en outre plus de 9% en poids d'ions de sodium.
